# EUROPEAN PATENT APPLICATION

(11) **EP 2 746 402 A1**
(43) Date of publication of application: **25.06.2014**
(21) Application number: 13158742.0
(22) Date of filing: 12.03.2013
(51) Int. Cl.: C12Q 1/37, C07K 7/00

(54) **Method of prognosis of Alzheimers disease and substrates for use therein**

(71) Applicant: Academisch Medisch Centrum, 1105 AZ Amsterdam (NL)
(72) Inventor: The designation of the inventor has not yet been filed
(74) Representative: de Pauw, Elmar Sebastian David

(57) **Abstract**

The invention relates to A substrate for detecting the activity of IDE, of formula F-P-Q
- wherein P represents a peptide comprising at least one cleavage site for Insulin Degrading Enzyme (IDE),
- wherein F represents a fluorescent dye which is attached to at least one amino acid residue of P,
- wherein Q represents a quencher substance being able to quench the fluorescent dye F, and being attached to at least one amino acid residue of P, and
- whereby F and Q are attached to amino acids residues on different sides of the cleavage site of P,
with the proviso that there is no cleavage site for Angiotensin Converting Enzyme (ACE) present in said peptide between the amino acids to which F and Q are attached.

SEQUENCE LISTING

<110> Academisch Medisch Centrum

<120> METHOD OF PROGNOSIS OF ALZHEIMERS DISEASE AND SUBSTRATES FOR USE
THEREIN

<130> INV-00413

<160> 10

<170> BiSSAP 1.2

<210> 1
<211> 40
<212> PRT
<213> Artificial Sequence

<220>
<223> Abeta

<400> 1
Asp Ala Glu Phe Arg His Asp Ser Gly Tyr Glu Lys His His Gln Lys
1               5                   10                  15
Leu Val Phe Phe Ala Cys Asp Val Gly Ser Asn Lys Gly Ala Ile Ile
            20                  25                  30
Gly Leu Met Val Gly Gly Val Val
        35                  40

<210> 2
<211> 8
<212> PRT
<213> Artificial Sequence

<220>
<223> Abeta cleavage region

<400> 2
His Gln Lys Leu Val Phe Phe Ala
1               5

<210> 3
<211> 19
<212> PRT
<213> Artificial Sequence

<220>
<223> Substrate 1

<400> 3
Ser Gly Tyr Glu Lys His His Gln Lys Leu Val Phe Phe Ala Cys Asp
1               5                   10                  15
Val Gly Ser

<210> 4
<211> 15
<212> PRT
<213> Artificial Sequence

<220>
<223> Substrate 2

<400> 4
Tyr Glu Lys His His Gln Lys Leu Val Phe Phe Ala Cys Asp Val
1               5                   10                  15

<210> 5
<211> 13
<212> PRT
<213> Artificial Sequence

<220>
<223> Substrate 3

<400> 5
Glu Lys His His Gln Lys Leu Val Phe Phe Ala Cys Asp
1               5                   10

<210> 6
<211> 25
<212> PRT
<213> Artificial Sequence

<220>
<223> Substrate 4

<400> 6
Arg His Asp Ser Gly Tyr Glu Lys His His Gln Lys Leu Val Phe Phe
1               5                   10                  15
Ala Cys Asp Val Gly Ser Asn Lys Gly
            20                  25

<210> 7
<211> 8
<212> PRT
<213> Artificial Sequence

<220>
<223> Prior art sequence

<400> 7
Lys Leu Val Phe Phe Ala Glu Gly
1               5

<210> 8
<211> 20
<212> DNA
<213> Artificial Sequence

<220>
<221> source
<222> 1..20
<223> /organism="Artificial Sequence"
      /note="forward primer"
      /mol_type="unassigned DNA"

<400> 8
ggacaggttt gcgcagtttt                                                   20


<210> 9
<211> 23
<212> DNA
<213> Artificial Sequence

<220>
<221> source
<222> 1..23
<223> /organism="Artificial Sequence"
      /note="reverse primer"
      /mol_type="unassigned DNA"

<400> 9
acagcgttca cctctctgtc ttt                                               23


<210> 10
<211> 12
<212> PRT
<213> Artificial Sequence

<220>
<223> Substrate 5

<400> 10
His Gln Lys Leu Val Phe Phe Ala Cys Asp Cys Asx
1               5                   10

## Description

### TECHNICAL FIELD OF THE INVENTION

The present invention relates generally to a method of diagnosis or prognosis of Alzheimer's disease (AD) and substrates for use therein. More specifically, the invention relates to a method of prognosticating AD by determining the capability of the Insulin Degrading Enzyme (IDE) to cleave Aβ proteins. The invention further relates to substrates suitable for determining the Alzheimer's disease status of a subject in a biological sample.

### BACKGROUND

Alzheimer's disease (AD) is a progressive brain disease with a huge cost to human patients and their families. AD is the most common form of dementia, a common term for memory loss and other cognitive impairments. The impact of AD is also a growing concern for governments due to the increasing number of elderly citizens at risk. No cure for AD is currently available, though a number of drug and non-drug based therapies for ameliorating the symptoms of AD are widely accepted. In general, drug treatments for AD are directed at slowing the progression of symptoms. While many such drug treatments have proven effective for many patients, success is directly correlated with detecting the presence of disease at its earliest stages.

Currently, no biochemical tests are known for the diagnosis of AD or for monitoring the progression of the disease. Certain publications have identified proteins or signatures that could be used as diagnostic tools for AD (see, e.g., Gomez Ravetti, M. et al., PLoS One, 3e3111 (2008); and Shaw, L. M. et al., Ann Neurol, 65, 403-13 (2009)). Most AD biomarker studies are focused on the quantitative changes in tau and A[beta] proteins and modifications of these proteins in the cerebrospinal spinal fluid (CSF) from AD patients. These studies have led to a consensus that an increase in both total tau and p-tau and a concomitant decrease in A[beta] 1-42 in CSF may be indicative of AD. However, these changes in t-tau, p-tau, A[beta] 1-42 are not specific indicators of AD and also occur in some other forms of dementia (N. Andreasen et al., Arch Neurol. 58, 373-379 (2001); Formichi, P. et al., J. Cell. Physiol. 208, 39-46 (2006); Lewczuk P, et al., Neurobiol. Aging. 25, 273-281 (2004); Sunderland T. et al., JAMA 289, 2094-2103 (2003); Bailey P. Can. J. Neurol. Sci. 34, Suppl. 1 S72-S76 (2007); Blennow K., J. Am. Soc. Exp. Neurotherapeutics. 1, 213-225 (2004)).

The global prevalence of AD is expected to grow from approximately 6 billion people in 2008 to 11 billion in 2030, and an urgent need exists to identify markers for early detection of AD and to monitor the effectiveness of potential new therapies. As the only body fluid in direct contact with the brain, cerebrospinal fluid (CSF) is a potentially rich source of molecular markers that may be able to provide early and specific indication of neurological disorders including sporadic AD.

### SUMMARY OF THE INVENTION

The present disclosure is based in part on the finding that Insulin Degrading Enzyme (IDE) degrades monomeric Aβ less efficiently at Braak stage I and II of sporadic AD (sAD), in contrast to non demented controls that are efficiently degrading Aβ. The capability of IDE of a subject to degrade amyloid-β proteins is therefore a biomarker for the detection of AD, especially at an early stage of the disease. In Kim et al. J Biol Chem. 2007 Mar 16;282(11):7825-32, it has been described that catalytic activity of IDE is decreased in subject suffering from familial Alzheimer's disease. The authors suggested that genetic linkage of affected families to chromosome 10 is driven by defects in IDE leading to dysfunction. As such genetic linkage is not expected in patients suffering from sporadic AD, it is very surprising that the catalytic activity of IDE is also compromised in biologal samples of patients at an early stage of sporadic AD, which were not associated with prior family history of the disease.

In a first aspect, the invention therefore provides a method of diagnosis or prognosis of sporadic Alzheimer's disease in a subject, comprising steps of determining the enzyme activity of IDE in a biological sample of a subject and diagnosing or prognosticating sporadic Alzheimer's disease based on said enzyme activity of IDE. Preferably, said biological sample is cerebrospinal fluid or blood.

In another aspect the invention relates to highly specific substrates for determining the enzyme activity of IDE which are therefore excellently suitable for detecting sporadic AD at an early phase in a biological sample of a subject.

Substrates which are suitable for determining the catalytic activity of IDE are described in EP1674580 A1. Herein, a compound is described having the formula F - P - Q
- wherein P represents a peptide comprising at least one cleavage site of the enzyme,
- wherein F represents a fluorophore which is attached to at least one amino acid residue of P,
- wherein Q represents a compound being able to statically quench the fluorophore F, and being attached to at least to one amino acid residue of P, and
- whereby F and Q are attached to amino acid residues on different sides of the enzyme cleavage site of P. EP1674580 A1, specifically discloses compounds having the amino acid sequence KLVFFAEG (SEQ ID NO:7). The drawback of this compound is that the inventors have found that by using this substrate for determining the catalytic activity of IDE present in a biological sample, a poor relation is obtained with prognosis of sporadic Alzheimer's disease.

Serendipously, the inventors have found that substrates wherein any of the amino acids AE in the recognition site KLVFFAE is mutated, substituted or deleted, substrates are obtained which are superior in detecting sAD. Without wishing to be bound by theory, the inventors believe that by mutating or deleting any of the amino acids AE, which are present in the wild type form of Aβ proteins directly flanking the carboxyl end of the VFF cleavage site for IDE (e.g. KLVFFAE) , the cleavage site for angiotensin converting enzyme (ACE) is disrupted and thereby improving the specificity of the substrate.

Therefore, in another aspect, the invention provides a substrate for detecting the activity of IDE, of formula F-P-Q
- wherein P represents a peptide comprising at least one cleavage site for Insulin Degrading Enzyme (IDE),
- wherein F represents a fluorescent dye which is attached to at least one amino acid residue of P,
- wherein Q represents a quencher substance being able to statically quench the fluorescent dye F, and being attached to at least one amino acid residue of P, and
- whereby F and Q are attached to amino acids residues on different sides of the cleavage site of P,
with the proviso that there is no cleavage site for Angiotensin Converting Enzyme (ACE) present in said peptide between the amino acids to which F and Q are attached.

In a preferred embodiment, said substrate is selected from the group consisting of: a protein having the amino acid sequence DAEFRHDSGYEKHHQKLVFFACDVGSNKGAIIGLMVGGVV (SEQ ID NO:1), a functional fragment thereof, a homologue thereof, and a chemical derivative of any of the protein, functional fragment and analogue as defined above. Preferably, said peptide has an amino acid sequence which is at least 70% homologous to SEQ ID NO: 1.

Preferably, said peptide comprises the amino acid sequence selected from the group consisting of: VFF, LVFF, KLVFF QKLVFF, HQKLVFF, QKLVFFA, HHQKLVFF and HQKLVFFA (SEQ ID NO:2).

In a preferred embodiment, said peptide according to the invention, comprises at least 10, more preferably at least 11, 12, 13, 14, 15, 16, 17, 18, 19, 20, 21, 22, 23, 24 or 25 amino acids.

In a preferred embodiment, said substrate according to the invention, comprises at its termini a chemical group, preferably a D amino acid, which protects against degradation by exopeptidases. In another embodiment, one or more amino acids of the substrate are chemically modified to make the substrate more stable. Preferably, said chemical modification comprises amidation and/or acetylation of the terminal amino acids of the substrate. This modification makes the resulting peptide more stable towards unwanted enzymatic degradation resulting from exopeptidases.

If the peptide is from an internal sequence of a protein, terminal amidation (C-terminus) or acetylation (N-terminus) will remove its charge and help it imitate its natural structure (amide, CONH2). In addition, this modification makes the resulting peptide more stable towards enzymatic degradation resulting from exopeptidases.

In a preferred embodiment, one or more D amino acids are incorporated in the terminal ends of said substrate. An advantage thereof is that both termini are protected from degradation, allowing only endopeptidases like IDE to degrade the substrate. Preferably the most terminal amino acids of both termini are D-amino acids.

In a preferred embodiment said substrate according to the invention has the amino acid sequence selected from the group consisting of: SGYEKHHQKLVFFACDVGS (SEQ ID No. 3), YEKHHQKLVFFACDV (SEQ ID No. 4), EKHHQKLVFFACD (SEQ ID No. 5) and RHDSGYEKHHQKLVFFACDVGSNKG (SEQ ID No. 6).

In a preferred embodiment, said quencher substance is selected from the group consisting of Dabcyl, QSY7, QSY33, Ferrocene and its derivatives, methyl viologen, and N,N'-dimethyl-2,9-diazopyrenium.

Preferably, said fluorescent dye is selected from the group consisting of fluorescein isothiocyanate (FITC) and its derivatives; Alexa 488, Alexa 532, Alexa 350, cy3, cy5, 6-joe, EDANS; rhodamine 6G (R6G) and its derivatives, Texas red, BODIPY FL, BODIPY FL/C3, BODIPY FL/C6, BODIPY 5-FAM, BODIPY TMR, and derivatives thereof, BODIPY R6G, BODIPY 564, and BODIPY 581.

In a highly preferred embodiment, said quencher substance is Dabcyl and said fluorescent dye is fluorescein.

Preferably, said quencher substance is positioned vis-à-vis said fluorescent dye, such that there are between 6-12 amino acids between said quencher substance and said fluorescent dye. Preferably, said quencher substance and said fluorescent dye are attached to the amino acids of position 12 and 22.

In a preferred embodiment of the method according to the invention, said activity is determined by incubating said biological sample with the substrate according to the invention, measuring the fluorescence of said substrate and determining the enzyme activity of IDE based on said fluorescence.

In another aspect the invention provides a method of determining the enzyme activity of IDE, comprising combining an amount of IDE and a substrate according to the invention, allow said IDE to degrade said substrate, and measuring the fluorescence of said substrate and determining the enzyme activity of IDE based on said fluorescence.

In a preferred embodiment of the method of diagnosis or prognosis of sporadic Alzheimer's disease according to the invention, said enzyme activity of IDE is determined according to the method of determining the enzyme activity of IDE according to the invention.

In a further aspect, the invention provides a method of selecting a compound or composition suitable for the treatment of Alzheimer's Disease comprising steps of:
- providing a sample comprising IDE, a compound or composition to be tested, and the substrate according to the invention,
- measuring the fluorescence of said substrate,
- determining the activity of IDE based on said fluorescence, and
- selecting the compound or composition based on an increased enzyme activity of IDE compared to a control sample.

### BRIEF DESCRIPTION OF THE DRAWINGS

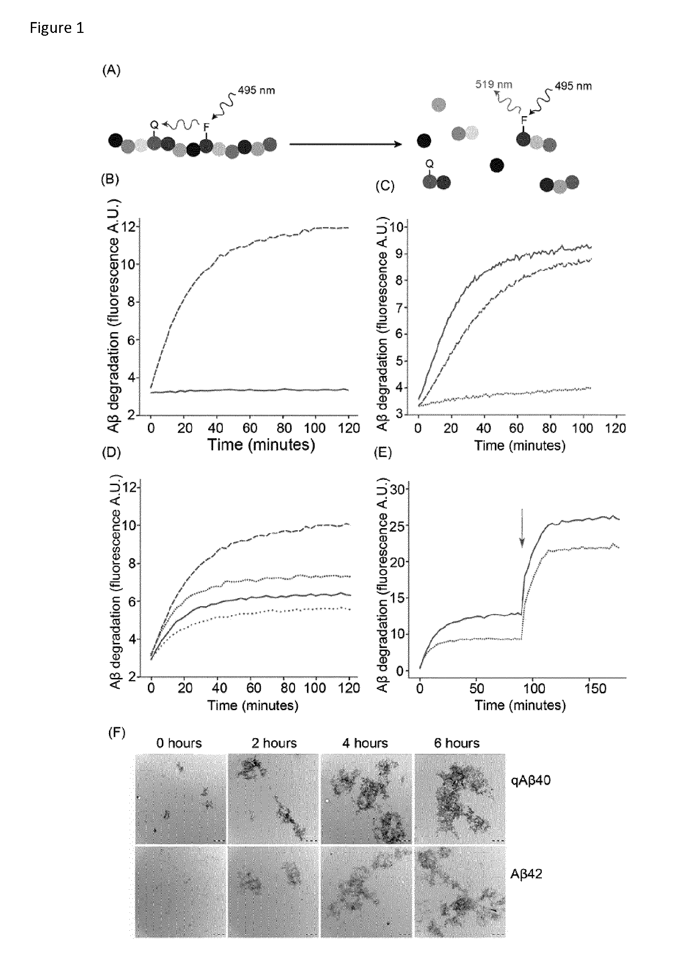

Figure 1(A) shows monomeric and oligomeric qAβ degradation. Fluorescence can only be detected upon degradation of the quenched Aβ (qAβ) peptide when the fluorescein group (F) is separated from the quencher group (Q). Figure 1(B) shows that qAβ is degraded in HEK293 cytosolic fractions (dashed line) but not in KMH buffer (solid line). Figure 1(C) shows qAβ degradation in HEK293 cytosolic fractions without inhibitor (solid line), pre-incubated with 100 µM phenantroline (dashed line) and pre-incubated with 200 µM bacitracin (dotted line). Figure 1(D) shows the degradation of qAβ that was pre-incubated at 37°C for 0 hour (dashed line), 2 hours (upper dotted line), 4 hours (solid line) or 6 hours (lower dotted line) in HEK293 cell lysates. Figure 1(E) shows that oligomeric Aβ does not impair IDE, as monomeric qAβ is as efficiently degraded in HEK293 cell lysates pre-incubated with monomeric qAβ (solid line) and with 2 hours pre-incubated qAβ (dotted line). The additional pool of monomeric qAβ was added when the fluorescence plateau level was reached (arrow). (F) Electron microscopy images showing increased oligomerisation with time of qAβ and synthetic Aβ₄₂ at 37°C.

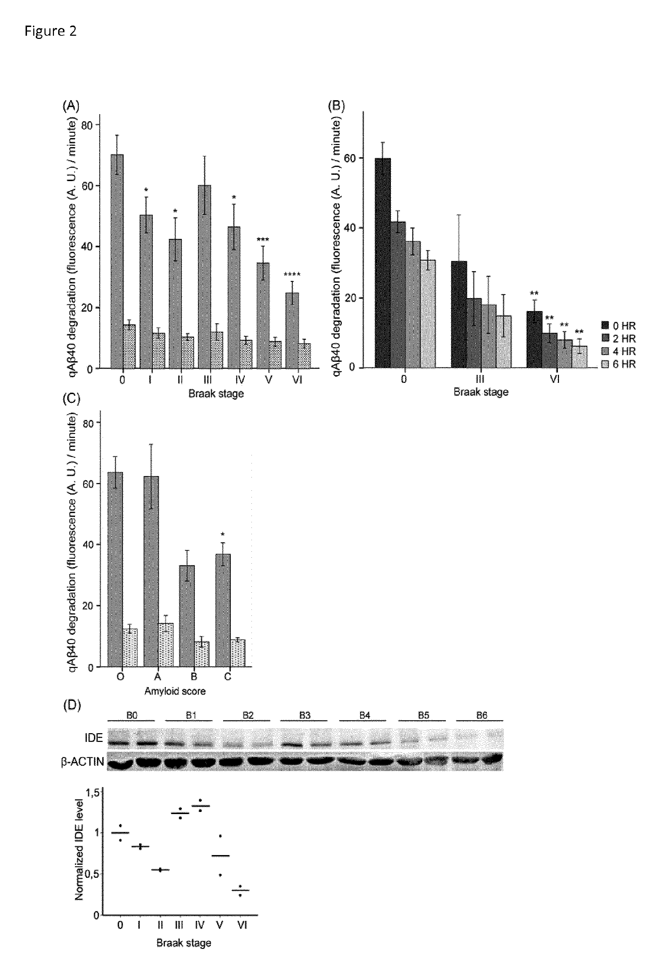

Figure 2 shows the qAβ degradation during the progression of sporadic AD. Figure 2(A) shows qAβ degradation in human hippocampal lysates at different Braak stages, without inhibitor (solid bars) and with bacitracin (dotted bars). qAβ degradation is significantly decreased in Braak stage I (p<0.05), II (p=0.01), IV (p<0.05), V (p<0.001) and VI (p<0.0001). Error bars represent standard error of the mean (SEM). Figure 2(B) shows the degradation of differently pre-incubated, oligomeric qAβ in human hippocampal lysates at Braak stage 0, III and VI. qAβ degradation is significantly decreased in Braak stage VI, at 0 hour (p<0.01), 2 hours (p<0.01), 4 hours (p<0.01) and 6 hours (p<0.01) of pre-incubation. Error bars represent SEM. Figure 2(C) shows the qAβ degradation in human hippocampal lysates at different amyloid scores. qAβ degradation is significantly decreased in amyloid score C (p<0.05). Error bars represent SEM. Figure 2(D) shows in the Upper panel: Western blot analysis of human hippocampal lysates at different Braak stages, showing a decrease of IDE with increasing Braak stage in sAD. In the lower panel of Figure 2(D) Western blot quantification of human hippocampal lysates at different Braak stages is shown.

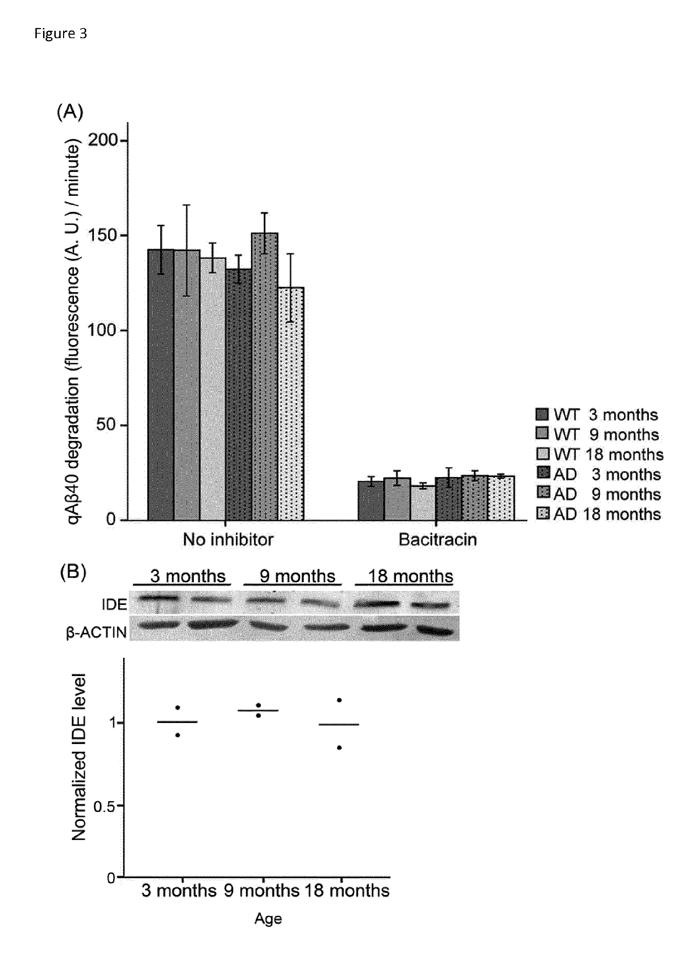

Figure 3 shows qAβ degradation during the progression of AD in the APPswePS1dE9 mice. Figure 3(A) shows qAβ degradation in mouse hippocampal lysates of both wild-type (solid bars) and APPswePS1dE9 mice (dotted bars) at 3, 9 and 18 months of age. Error bars represent standard error of the mean (SEM). Figure 3(B) Upper panel shows the Western blot analysis of APPswePS1dE9 mice hippocampal lysates at 3, 9 and 18 months of age, stained for IDE and β-actin (loading control). In the lower panel of Figure 3(B), Western blot quantification of APPswePS1dE9 mice hippocampal lysates at different ages is shown.

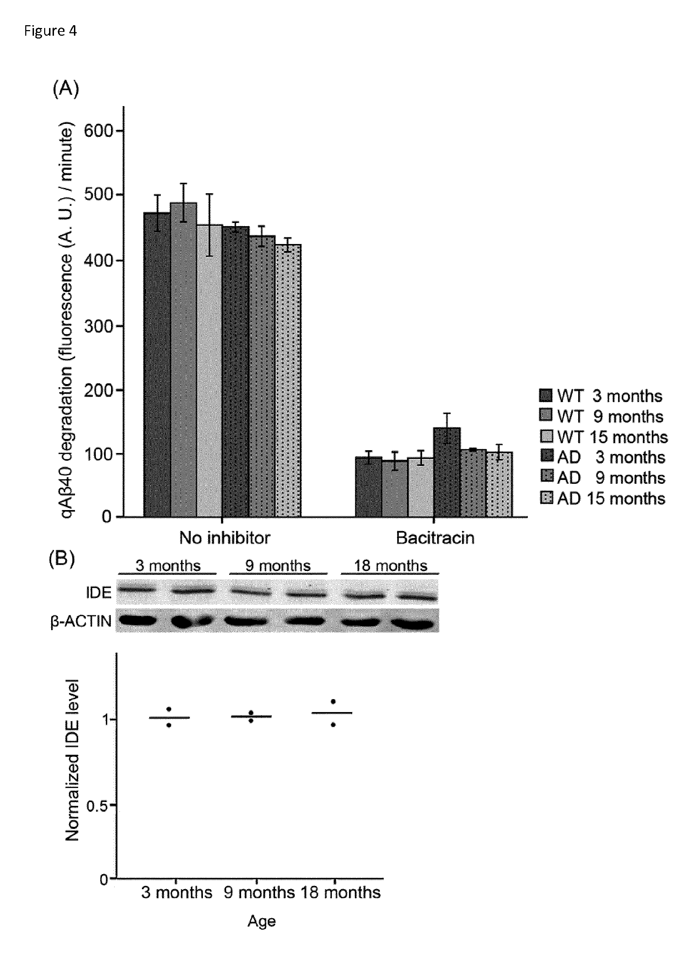

Figure 4 shows qAβ degradation during the progression of AD in the 3xTg-AD mice. Figure 4(A) shows qAβ degradation in mouse hippocampal lysates of both wild-type (solid bars) and 3xTg-AD mice (dotted bars) at 3, 9 and 15 months of age. Error bars represent standard error of the mean (SEM). Figure 4(B), upper panel shows the Western blot analysis of 3xTg-AD mice hippocampal lysates at 3, 9 and 15 months of age, stained for IDE and β-actin (loading control). In the lower panel of Figure 4(B), the Western blot quantification of 3xTg-AD mice hippocampal lysates at different ages is shown.

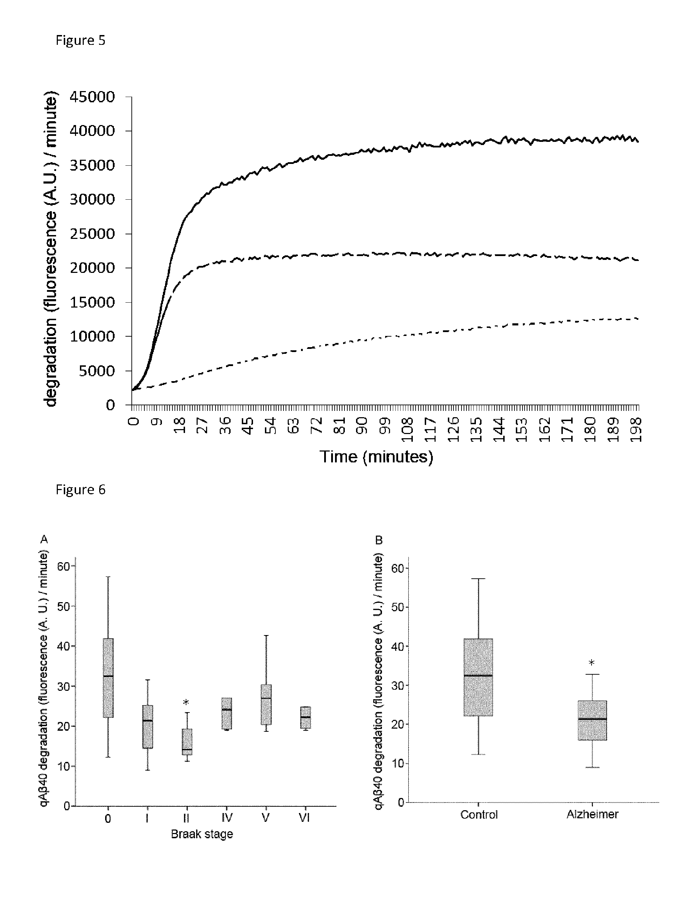

Figure 5 shows the degradation of the 12-mer HQKLVFFACDCB peptide as a monomeric form (solid line), an oligomeric form, added after 3 HR incubation in PBS (dashed line) and the monomeric form in the presence of Bacitracin (dotted line).
Figure 6 shows qAβ40 degradation in human post mortem Cerebrospinal fluid. Figure 6(A) shows the degradation of the qAβ40 peptide in human post mortem CSF obtained from patients at different Braak stages and healthy controls. qAβ40 degradation in significantly decreased at Braak stage II (* p<0.05). Figure 6(B) shows the degradation of the qAβ40 peptide in human post mortem pooled CSF obtained from Alzheimer patients (Braak stage I to VI) and healthy controls. qAβ40 degradation in significantly decreased in pooled CSF obtained from Alzheimer patients (* p<0.05).

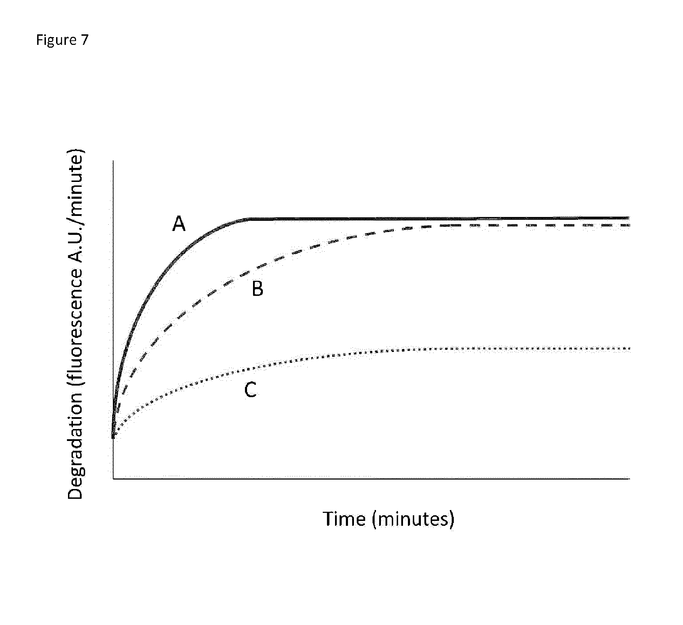

Figure 7 shows the effect of aggregation on the sensitivity of a substrate of the invention to detect IDE activity. Line (A) shows the development in time of the fluorescence of a substrate of the invention in the presence of IDE with a normal enzyme activity. When high IDE activity is present (as in healthy control persons) the substrate is rapidly degraded in time, leading to a fast increase in fluorescence and a high plateau level, indicating that the substrate was degraded fast enough to prevent its own aggregation during the assay. Line (B) shows the effect of less IDE activity (e.g. IDE from a subject in Braak I phase) when only looking at the speed of substrate degradation. With less IDE activity, the degradation rate of the substrate is reduced, resulting in an increased half-life of the substrate and therefore a longer time needed to reach the same plateau phase when compared to (A). From this, it can be concluded that the degradation rate of substrate is decreased in comparison to the results shown in line A, resulting in a slower increase in time. Due to the slower degradation rate, the substrate also has time to start oligomerizing during the assay. This results in less monomeric substrate that can be degraded by IDE, resulting in a lower plateau phase too (C). The slower degradation of the substrate allows non-degraded substrate to oligomerize in time, which prevents degradation by IDE. As a result, less substrate is degraded in samples with lower IDE activity, leading to a lower end plateau level of fluorescence (C). The substrate is therefore degraded at a slower rate and in lower quantities in samples with lower IDE activity. A substrate of the invention with the capacity to aggregate will therefore have a higher sensitivity for the detection of IDE activity than a substrate which does not aggregate.

### DETAILED DESCRIPTION OF THE INVENTION

### Definitions

As used herein, the terms "Alzheimer's disease" and "AD" refer to a neurodegenerative disorder and encompasses familial Alzheimer's disease and sporadic Alzheimer's disease. The term "familial Alzheimer's disease" refers to Alzheimer's disease associated with genetic factors (i.e., demonstrates inheritance) while "sporadic Alzheimer's disease" refers to Alzheimer's disease that is not associated with prior family history of the disease. Symptoms indicative of Alzheimer's disease in human subjects typically include, but are not limited to, mild to severe dementia, progressive impairment of memory (ranging from mild forgetfulness to disorientation and severe memory loss), poor visuo-spatial skills, personality changes, poor impulse control, poor judgement, distrust of others, increased stubbornness, restlessness, poor planning ability, poor decision making, and social withdrawal. In severe cases, patients lose the ability to use language and communicate, and require assistance in personal hygiene, eating and dressing, and are eventually bedridden. Hallmark pathologies within brain tissue include extracellular neuritic β-amyloid plaques, neurofibrillary tangles, neurofibrillary degeneration, granulovascular neuronal degeneration, synaptic loss, and extensive neuronal cell death.

The term "Amyloid-β protein" refers to any peptide produced by proteolytic processing of the APP gene product, especially peptides which are associated with amyloid pathologies, including Aβτ-39, Aβwo, Aβτ-41, Aβi42, and Aβws.

As used herein, the terms "β amyloid," "amyloid-β," and "Aβ" are synonymous. As used herein, the amyloid-β protein includes naturally occurring human wild type amyloid beta peptides. Wild type amyloid beta peptides include amyloid beta 1-38, amyloid beta 1-39, amyloid beta 1-40, amyloid beta 1-41, amyloid beta 1-42, and amyloid beta 1-43.

The structure and sequence of Aβ peptides of various lengths are well known in the art. Such peptides can be made according to methods known in the art (e.g., Glenner and Wong, Biochem Biophys. Res. Comm. 129: 885-890, 1984; Glenner and Wong, Biochem Biophys. Res. Comm. 122: 113 1-1135, 1984). In addition, various forms of the peptides are commercially available.

Rather than using the naturally occurring amyloid-β protein, functional derivatives of these peptides can be used. By "functional derivative" is meant a fragment, variant, analog, or chemical derivative of the amyloid-β peptides, which terms are defined below. A functional derivative retains at least its capability of being cleaved by the IDE. This capability can be tested by combining said fragment with a functional amount of IDE and allow said fragment to be cleaved. Subsequently, the fragment size may be determined, for example using western blotting or Mass spectometry.

The term "fragment" as used herein refers to a fragment of an Aβ protein which can be degraded by IDE and therefore is still functional as a specific substrate for IDE.

A "homologue" of the amyloid-β peptide refers to any peptide having a high degree of sequence identity with amyloid-β protein. As used herein, a homologue includes natural mutants and analogs of the amyloid-β protein. The definition of these terms is set below.

A "natural mutant" of the amyloid-β peptide refers to a molecule which is substantially similar to either the entire peptide or a fragment thereof. In particular, it refers to naturally occurring mutant amyloid beta peptides. Amyloid beta mutations include A2T, H6R, D7N, A21G, E22G (Arctic), E22Q (Dutch), E22K (Italian), D23N (Iowa), A42T, and A42V. These mutations may optionally be present in any of the amyloid beta peptides 1-38, 1-39, 1-40, 1-41, 1-42, and 1-43. Variant peptides can be conveniently prepared by direct chemical synthesis of the variant peptide, using methods well known in the art.

An "analog" of the amyloid-β peptide refers to a non-human molecule or to a non-human homolog which is substantially similar to the entire molecule or to a functional fragment thereof which is still functional as a specific substrate for IDE. It also includes non-naturally occurring variants having a high similarity with amyloid-β protein.

A "chemical derivative" of the amyloid-β peptide contains additional chemical moieties not normally part of the amyloid-β amino acid sequence. Covalent modifications of the amino acid sequence are included within the scope of this invention. These modifications may be introduced into the amyloid-β peptides by reacting targeted amino acid residues from the peptide with an organic derivatizing agent that is capable of reacting with selected side chains or terminal residues. Alternatively, D-amino acids may be incorporated in the amino acid sequence.

The term "Insuline Degrading Enzym" (IDE) as used herein refers to Insulin-degrading enzyme (IDE), which is a Zn2+-metalloprotease with a molecular weight of 113 kDa. The active site signature sequence of IDE consists of His-Glu-aa-aa-His (HEXXH) in which the two histidines coordinate the binding of the zinc ion and the glutamate plays an essential role in catalysis. IDE is ubiquitously expressed with its highest expression in the liver, testes, muscle and brain. IDE is abundant in the cytosol and peroxisomes and is also found in the rough endoplasmatic reticulum.

The gene encoding IDE is located on chromosome 10q23-q25 in humans. It spans approximately 120 kb and contains 24 exons. The coding sequence is highly conserved during evolution from E. coli, to Drosophila, to human.

IDE has been shown to play role in the degradation and clearance of insulin in vivo. Furthermore, IDE shows a degradation potential for some peptidic hormones and for amyloid-β peptide. Overexpression of IDE in cells in culture has been found to increase the rate of insulin degradation.

It has been shown that IDE is capable of degrading amyloid-β (Aβ). Aβ is neurotoxic and its accumulation results in amyloid fibril formation and the generation of senile plaques, the hallmark of Alzheimer's disease. It was suggested that IDE is involved in the clearance of Aβ from the brain and cerobrospinal fluid (CSF) and thereby preventing the formation of senile plaques. The mapping of the IDE gene to chromosome 10q23-q25 made it a candidate gene for the Alzheimer disease-6 locus.

The DNA sequence of human IDE (hIDE) has the mRNA nucleic acid sequence according to NM_001165946.1, and the protein has the amino acid sequence NP_001159418.1.

The term "fluorescent dye" as used herein means fluorescent dyes of the like, which are generally used for the determination or detection of nucleic acids by labeling nucleic acid probes. Illustrative of such fluorescent dyes are fluorescein and derivatives thereof [for example, fluorescein isothiocyanate (FITC) and its derivatives]; Alexa 488, Alexa 532, Alexa 350, cy3, cy5, 6-joe, EDANS; rhodamine 6G (R6G) and its derivatives [for example, tetramethylrhodamine (TMR), tetramethylrhodamine isothiocyanate (TMRITC), x-rhodamine, Texas red, "BODIPY FL" (trade name, product of Molecular Probes, Inc. (Eugene, Oreg., U.S.A.), "BODIPY FL/C3" (trade name, product of Molecular Probes, Inc.), "BODIPY FL/C6" (trade name, product of Molecular Probes, Inc.), "BODIPY 5-FAM" (trade name, product of Molecular Probes, Inc.), "BODIPY TMR" (trade name, product of Molecular Probes, Inc.), and derivatives thereof (for example, "BODIPY TR" (trade name, product of Molecular Probes, Inc.), "BODIPY R6G" (trade name, product of Molecular Probes, Inc.), "BODIPY 564" (trade name, product of Molecular Probes, Inc.), and "BODIPY 581" (trade name, product of Molecular Probes, Inc.)]. Among these, FITC, EDANS, Texas red, 6-joe, TMR, Alexa 488, Alexa 532, " BODIPY FL/C3" and "BODIPY FL/C6" are preferred, with EDANS, Texas red, FITC, TMR, 6-joe, "BODIPY FL/C3" and "BODIPY FL/C6" being more preferred.

The term "quencher substance" as used herein refers to a substance which acts on the above-described fluorescent dye and inhibits or quenches emission of fluorescence from the fluorescent dye. Illustrative are Dabcyl, "QSY7" (Molecular Probes), "QSY33" (Molecular Probes), Ferrocene and its derivatives, methyl viologen, Blackberry quenchers for red fluorophores, such as TAMRA, Cy3/Cy5 (Berry & Associates, Inc., Dexter, USA), Dansyl, DNP (Biopeptek, Inc., Malvern, USA), and N,N'-dimethyl-2,9-diazopyrenium, with Dabcyl and the like being preferred.

As used herein, the term "amino acid" refers to the aminocarboxylic acids that are components of proteins and peptides. The amino acid abbreviations are as follows: A (Ala); C (Cys); D (Asp); E (Glu); F (Phe); G (Gly); H (His); I (Iso); K (Lys); L (Leu); M (Met); N (Asn); P (Pro); Q (Gin); R (Arg); S (Ser); T (Thr); V (Val); W (Trp), and Y (Tyr).

As used herein, the term "homologous" or means either i) a protein or peptide that has an amino acid sequence that is substantially similar (i.e., at least 70, 75, 80, 85, 90, 95, or 98%) to the sequence of a given original protein or peptide and that retains a desired function of the original protein or peptide or ii) a nucleic acid that has a sequence that is substantially similar (i.e., at least 70, 75, 80, 75, 90, 95, or 98%) to the sequence of a given nucleic acid and that retains a desired function of the original nucleic acid sequence. In all of the embodiments of this disclosure and disclosure, any disclosed protein, peptide or nucleic acid can be substituted with a homologous or substantially homologous protein, peptide or nucleic acid that retains a desired function.

When a subunit position in both of the two molecules is occupied by the same monomeric subunit, e.g., if a position in each of two peptide molecules is occupied by valine, then they are homologous at that position. The homology between two sequences is a direct function of the number of matching or homologous positions, e.g., if half (e.g., five positions in a polymer ten subunits in length) of the positions in two compound sequences are homologous then the two sequences are 50% homologous, if 90% of the positions, e.g., 9 of 10, are matched or homologous, the two sequences share 90% homology. By way of example, the amino acid sequences KLVFFA and DLGFFV share 50% homology.

The determination of percent identity between two amino acid sequences can be accomplished using a mathematical algorithm. For example, a mathematical algorithm useful for comparing two sequences is the algorithm of Karlin and Altschul (1990, Proc. Natl. Acad. Sci. USA 87:2264-2268), modified as in Karlin and Altschul (1993, Proc. Natl. Acad. Sci. USA 90:5873-5877). This algorithm is incorporated into the NBLAST and XBLAST programs of Altschul et al. (1990, J. Mol. Biol. 215:403-410), and can be accessed, for example, at the BLAST site of the National Center for Biotechnology Information (NCBI) world wide web site at the National Library of Medicine (NLM) at the National Institutes of Health (NIH). BLAST protein searches can be performed with the XBLAST program (designated "blastn" at the NCBI web site) or the NCBI "blastp" program, using the following parameters: expectation value 10.0, BLOSUM62 scoring matrix to obtain amino acid sequences homologous to a protein molecule described herein.

To obtain gapped alignments for comparison purposes, Gapped BLAST can be utilized as described in Altschul et al. (1997, Nucleic Acids Res. 25:3389-3402). Alternatively, PSI-Blast or PHI-Blast can be used to perform an iterated search which detects distant relationships between molecules (id.) and relationships between molecules which share a common pattern. When utilizing BLAST, Gapped BLAST, PSI-Blast, and PHI-Blast programs, the default parameters of the respective programs (e.g., XBLAST and NBLAST) can be used as available on the website of the National Center for Biotechnology Information of the National Library of Medicine at the National Institutes of Health.

The percent identity between two sequences can be determined using techniques similar to those described above, with or without allowing gaps. In calculating percent identity, typically exact matches are counted.

As used herein, the term "exopeptidase" refers to a hydrolase enzyme that removes terminal amino acids of a peptide or protein by cleaving peptide bonds.

Throughout the application, all references to a particular amino acid position by number (e.g., position 28) refer to the amino acid at that position in SEQ ID NO:1 or the corresponding amino acid position in any functional derivative, fragment, variant, analog, chemical derivative thereof. For example, a reference herein to "position 28" would mean the corresponding position 27 for an analog in which the first amino acid of SEQ ID NO:1 has been deleted. Similarly, a reference herein to "position 28" would mean the corresponding position 29 for an analog in which one amino acid has been added before the N-terminus of SEQ ID NO:1. As used herein an "amino acid modification" refers to (i) a substitution or replacement of an amino acid of SEQ ID NO:1 with a different amino acid (naturally-occurring or coded or non-coded or non-naturally-occurring amino acid), (ii) an addition of an amino acid (naturally-occurring or coded or non-coded or non-naturally-occurring amino acid), to SEQ ID NO:1 or (iii) a deletion of one or more amino acids of SEQ ID NO: 1.

### Preferred embodiments

### Peptide substrate for detecting the activity of Insulin-degrading enzyme

The invention provides a substrate for detecting the activity of IDE, of formula F-P-Q
- wherein P represents a peptide comprising at least one cleavage site for Insulin Degrading Enzyme (IDE),
- wherein F represents a fluorescent dye which is attached to at least one amino acid residue of P,
- wherein Q represents a quencher substance being able to quench the fluorescent dye F, and being attached to at least one amino acid residue of P, and
- whereby F and Q are attached to amino acids residues on different sides of the cleavage site of P,
with the proviso that there is no cleavage site for Angiotensin Converting Enzyme (ACE) present in said peptide between the amino acids to which F and Q are attached. The substrate according to the invention serves as an easily quantifiable substrate for IDE, to enable measuring the enzyme activity level based on the fluorescence level of the spliced product. In order to be able to quantify the amount of cleavage of the protein of the invention by IDE, it is required that in one region flanking the cleavage site for IDE the fluorescent dye is present and that in the other flanking region the quencher substance is present. When the substrate according to the invention is in the unspliced form, said quencher substance prevents fluorescence by said fluorescent dye. This quenching effect is removed when the substrate of the invention is spliced, resulting in separate fragments having a fluorescent dye or a quenching substance, which are not close enough to interact. As a result of the cleavage, the fragments start to fluoresce.

The substrate according to the invention comprises a splice site for IDE, which is preferably in the middle of the substrate. The splice site is surrounded by amino acids which form also a critical region for aggregation. This cleavage site for IDE is crucial for the functionality of the substrate of the invention, because it confers the specificity of the substrate of the invention for the enzyme IDE. This cleavage site comprises at least the amino acid sequence VFF, because this stretch serves as recognition moiety for IDE and cleavage by IDE occurs in this sequence. Therefore, said peptide comprises at least the amino acids having the sequence VFF. Without wishing to be bound by theory, it is believed that splicing may occur in the peptide bond following each of the amino acids of the VFF sequence. As an example, a peptide having the amino acid sequence

DAEFRHDSGYEKHHQKLVFFACDVGSNKGAIIGLMVGGVV may be cleaved by IDE into the following fragment pairs:
DAEFRHDSGYEKHHQKLV and FFACDVGSNKGAIIGLMVGGVV,
DAEFRHDSGYEKHHQKLVF and FACDVGSNKGAIIGLMVGGVV, or
DAEFRHDSGYEKHHQKLVFF and ACDVGSNKGAIIGLMVGGVV

More preferably, said peptide comprises LVFF and even more preferably at least KLVFF, because these sequences are recognized by IDE with a higher degree of specificity. In addition, the substrate of the invention comprises two regions flanking said splice site, which become separate fragments upon splicing by IDE. Each of these regions comprise either a quencher substance or a fluorescent dye. At least one region comprises said quencher substance and the other region comprises said fluorescent dye. The quenching substance may be in the region on the N-terminus of the substrate of the invention or on the C terminus. It is understood that if the quenching substance is present in the region on the C terminus, then a fluorescent substance is present in the other region on the N terminal side of the protein, and vice versa.

In principle, any peptide substrate comprising said cleavage site for IDE may be used. Any amyloid-β protein or functional derivative, fragment, variant, analog, chemical derivative thereof as defined herein may be used as the basis for the preparation of substrate according to the invention, as long as it can be spliced by IDE.

In a preferred embodiment, said peptide comprises the amino acid sequence having at least 5, more preferably 6, 7, 8 or all consecutive amino acids of the amino acid sequence HHQKLVFFA (SEQ ID NO:2). An advantage of more amino acids of this sequence is that it improves the specificity. Good results may be obtained when said recognition site comprises the amino acid sequence selected from the group consisting of: QKLVFF, KLVFFV, QKLVFFV, HQKLVFF, HHQKLVF, QKLVFFA,

HHQKLVFF and HQKLVFFA (SEQ ID NO:2).

Preferably, said substrate has an amino acid sequence which is identical to at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% to the following sequence:

DAEFRHDSGYEKHHQKLVFFACDVGSNKGAIIGLMVGGVV (SEQ ID No. 1)
1234567890123456789012345678901234567890(position)

Said substrate according to the invention may vary in length as long as its functionality is retained. The length of the substrate should preferably not be longer than 41 amino acids, as this leads to aggregation during synthesis. Also fragments of the substrate according to the invention which can be degraded by IDE may be used.

Relatively short substrates, for example shorter fragments of the amyloid-β protein or fragments of a functional derivative, fragment, variant, analog, chemical derivative of said amyloid-β protein may also be used. However, fragments which are shorter than 10, 11, 12, 13, 14, 15, 16, 17, 18, 19, 20, 21, 22, 23, 24 or 25 amino acids should preferably not be used, as such short fragments are less aggregation-prone. For this reason, aggregation cannot be used as a parameter for IDE activity. Therefore, in a more preferred embodiment, said fragments are capable of aggregating. This greatly facilitates the method according to the invention, because it results not only in slower degradation of said fragments, but also results in a lower fluorescence after the reaction is concluded. As a result, short substrates result in a lower sensitivity in the detection of IDE activity. The relation between aggregation capability of a substrate according to the invention and its sensitivity for determining ID activity is shown in Figure 7.

Any method known in the art may be used to establish whether an amyloid-β protein, functional derivative, fragment, variant, analog, chemical or derivative is capable of aggregation. For instance, said protein or fragment etc. may be visualized by transmission electron microscopy to determine whether they form oligomers and fibers in time. Alternatively, the aggregation capability of a peptide may be determined by incubating the peptide with IDE and subsequently determining the degradation decrease. As IDE only cleaves monomeric substrates, a decrease in degradation can be determined in case the peptide is aggregated. It is also possible to determine the aggregation capability of a peptide by incubating the peptide with fluorescent compounds like CongoRed or Thioflavine S. Aggregated peptides may be visualized by fluorescent microscopy.

The distance between the fluorescent dye and the quenching substance is very important for the functioning of the protein according to the invention. When the distance is too large, the fluorescent dye and the quenching substance are unable to interact. The distance between the amino acids at the positions where the protein of the invention is labeled with the fluorescent dye and the quencher substance is preferably between 6 and 12, more preferably between 8 and 12, in terms of the number of amino acids, including the amino acids labeled with the fluorescent dye and quenching substance. For example, if a quencher substance is position at amino acid 10 and a fluorescent dye at amino acid 22, the distance between the quenching substance and the fluorescent dye is 12 amino acids.

The distance between the amino acids depends strongly upon the base sequence of the probe, the fluorescent dye and quencher substance to be used for modification, the lengths of linkers adapted to bind them to the amino acid, and the like. It is, therefore, difficult to fully specify the amino acid-to-amino acid distance. It is to be noted that the above-described acid-to-amino acid distances are merely general examples and the distance between the bases includes many exceptions.

In a preferred embodiment, said distance between the fluorescent dye and the quenching substance is 10, 11 or 12 amino acids. Preferably, said fluorescent dye and said quencher substance are at position 22 and at position 12 of SEQ ID. NO:3 or at the comparable positions within a homolog of the protein according to the invention. In a highly preferred embodiment, said substrate comprises the amino acid sequence HQKLVFFA, wherein the amino acids flanking directly the amino acid sequence HQKLVFFA are labeled on the one side with a quencher substance and on the other side with a fluorescent dye, for example as indicated with the following formula:
[Q]HQKLVFFA[F] or [F]HQKLVFFA[Q],
wherein [Q] indicates an amino acid labeled with a quenching substance and [F] indicates an amino acid labeled with a fluorescent dye.

In a more preferred embodiment, said amino acid flanking the N- terminal side of the amino acid sequence HQKLVFFA is a His (H). In another preferred embodiment, said amino acid flanking the C- terminal side of the amino acid sequence HQKLVFFA is a Cys (C).

A highly preferred embodiment of the substrate of the invention includes a Aβ40 peptide having the amino acids sequence according to SEQ ID NO:1 or a homologous protein thereof, containing a fluorescein-labeled amino acid such as cysteine at position 22 and a quencher-labeled amino acid such as histidine at position 12. It is highly preferred that such fragments comprise the amino acid sequence HQKLVFFA, preferably flanked on one side by an aminoacid with a quenching group, and on the other side by an amino acid with a fluorophore: [Q]HQKLVFFA[F] or [F]HQKLVFFA[Q]. Optionally, said substrate may further comprise additional amino acids at the N-terminus and/or C-terminus, for example as indicated with the following formula:
xyzE(Q)HHQKLVFFA(F)Dxyz or xyzE(F)HHQKLVFFA(Q)Dxyz
wherein [Q] indicates an amino acid labeled with a quenching substance and [F] indicates an amino acid labeled with a fluorescent dye. "xyz" indicate a variable number of random amino acids.

The substrate of the invention may be synthesized according to any method known to the skilled person, for example such as described in Reits et al., 2004.

In a preferred embodiment, said substrate according to the invention, comprises at its termini a chemical group, preferably a D amino acid, which protects against degradation by exopeptidases. In another embodiment, one or more amino acids of the substrate are chemically modified to make the substrate more stable. Preferably, said chemical modification comprises amidation and/or acetylation of the terminal amino acids of the substrate. This modification makes the resulting peptide more stable towards enzymatic degradation resulting from exopeptidases.

In a preferred embodiment, said modification comprises amidation of the C terminus and/or acetylation of the N-terminus. This modification makes the resulting peptide more stable towards enzymatic degradation resulting from exopeptidases.

In a preferred embodiment, one or more D amino acids are incorporated in the terminal ends of said amyloid-β protein, analogue or functional fragment. An advantage thereof is that both termini are protected from degradation, allowing only endopeptidases like IDE to degrade the qAb. Preferably the most terminal amino acids of both termini are D-amino acids.

### Method of diagnosis or prognosis of Alzheimer's disease

The inventors have surprisingly found that the enzyme activity of IDE is impaired already at an early stage during the development of Alzheimer's disease. When the ability to degrade qAβ (qAβ = quenched Amyloid-β, which is a substrate according to the invention) was determined for each Braak stage (Fig. 2A, solid bars), a strong decrease in qAβ degradation rate was already observed in Braak stage I (t-test, two-tailed, p=0.03), which further decreased during the progression to Braak stage II (t-test, two-tailed, p=0.01). In Braak stage III the ability to degrade qAβ was partially restored, but still decreased, only to decrease again in Braak stage IV (t-test, two-tailed, p=0.02) and further decrease as a function of progression of Braak stage (Braak V: t-test, two-tailed, p=0.0005, Braak VI: t-test, two-tailed, p=0.000, t-test). In Braak VI, qAβ degradation rate decreased with more than 65% compared to Braak 0. When bacitracin was added to hippocampal lysates prior to addition of the qAβ, the ability to degrade qAβ was strongly reduced in all Braak stages upon IDE inhibition (Fig. 2A, dotted bars). This indicates that IDE is also the main protease responsible for Aβ clearance in human hippocampal lysates and suggests that IDE is impaired in sporadic Alzheimer's disease.

Since changes in Aβ degradation can already be monitored during the earliest stages of the disease, this would provide possibilities to diagnose Alzheimer's disease before the appearance of symptoms and the development of plaque pathology.

Therefore, the invention relates in another aspect to a method of diagnosis or prognosis of Alzheimer's disease in a subject, comprising steps of determining the enzyme activity of IDE. The activity of IDE present in a biological sample from the subject in a biological sample may be determined using any means known in the art. A suitable methods for determining the enzyme activity of IDE includes for example, using the fluorometric assay as described in James Scott Miners, Patrick Gavin Kehoe, Seth Love, , J of Neuroscience Methods, Vol. 169, Issue 1, 30 March 2008, Pages 177-181. When the capacity of the IDE to digest an Aβ protein is reduced when compared to the activity of IDE from a healthy control, there is an increased chance that the patient will develop Alzheimer's disease or already suffers from Alzheimer's disease. Preferably, the IDE activity is determined using the method according to the invention. In another preferred embodiment, said IDE activity is determined using a substrate as described in EP1674580A1, wherein said IDE activity is determined in the presence of an ACE inhibitor. An advantage thereof is that the presence of ACE inhibitors improve the specificity of the method. ACE inhibitors are known in the art and include, for example lisinopril (CAS number 83915-83-7).

Said biological sample is a sample which contains IDE in a detectable concentration. Therefore, it is preferred to use cerebrospinal fluid (CSF) samples, because these samples are easily obtainable and contain IDE in suitable amounts. In another preferred embodiment, said biological sample comprises peripheral blood or serum.

In a more preferred embodiment of the method of the invention, the IDE activity in a sample of a subject is quantified by incubating said biological sample with the substrate according to the invention, measuring the fluorescence of said protein or functional part thereof and determining the enzyme activity of IDE based on said fluorescence.

Preferably, the degradation of the protein or functional part thereof according to the invention is analyzed using a fluorometer.

In a preferred embodiment, the method according to the invention is a method of prognosis and is used to test subjects in an early stage, before the clinical manifestations of sporadic Alzheimer's disease. In such early stages, there are currently no tests which may be used to prognosticate the disease. For example, mental tests, such as the Mini Mental State Examination (MMSE) test cannot be related to the lowest Braak stages (see Neurobiology of Aging 33 (2012) Preclinical AD Workgroup staging: pathological correlates and potential challenges, page 622.e9, Figure 6). Therefore, in a preferred embodiment, said subject is a patient who has a normal MMSE score.

In a preferred embodiment, the method is combined with another biomarker for AD which is suitable for detecting AD at an early stage, preferably a biomarker which correlates with Braak I and/or Braak II. Such biomarker may comprise AD biomarkers including cerebrospinal fluid markers (amyloid, tau, phospho-tau, isoprostanes, and others) and imaging modalities such as volumetric magnetic resonance imaging (MRI), diffusion tensor imaging, functional MRI, metabolic positron emission tomography (PET), and amyloid ligand-PET. More preferably, said another biomarker comprises the MRI STructural Abnormality iNDex (STAND), which is described in Neurobiology of Aging 33 (2012) 622.e1- 622.e16, and is specifically illustrated on page S51, figure 3; "MRI STructural Abnormality iNDex (STAND) score versus Braak stage".

In a preferred embodiment, said another biomarker does not indicate a difference with a healthy control, while the IDE activity, based on the degradation of the substrate of the invention, is lower than in a healthy control. This combination is indicative of a subject developing AD and said subject is probably in Braak I or II. In another preferred embodiment, said another biomarker does indicate a difference with a healthy control, and the IDE activity, based on the degradation of the substrate of the invention, is also lower than in a healthy control. This combination is indicative of a subject developing AD and is probably in Braak IV, V or VI. In yet another preferred embodiment, said another biomarker indicates a difference with a healthy control, while the IDE activity, based on the degradation of the substrate of the invention, is not lower than in a healthy control. Such combination is indicative of a subject developing AD and is in Braak III.

### Method for screening and/or selecting a compound or composition suitable for the treatment of Alzheimer's Disease

In another aspect, the invention provides a method for screening and/or selecting a compound or composition suitable for the treatment of Alzheimer's Disease comprising steps of:
- providing a sample comprising IDE, a compound or composition to be tested, and the substrate according to the invention,
- measuring the fluorescence of said substrate,
   determining the activity of IDE based on said fluorescence,
- selecting the compound based on a changed enzyme activity of IDE compared to a control sample.

In this method, a compound or composition may be tested which directly interacts with IDE or the substrate. Such compounds or compositions are suitable in the treatment of Alzheimer's disease.

It is also possible to use the method for selecting compounds which interact with factors within neuronal cells and indirectly exert an influence on the degradation of Amyloid-β peptides. For example, a compound or composition may induce a chaperone that can re-solubilize Amyloid-β peptides. Alternatively, said compound or composition may result in increased expression or translation of IDE, post-translational modifications of IDE, improved complex formation of IDE, increased half-life of IDE, increased catalytic activity of IDE. Therefore, in another embodiment, said sample comprising IDE further comprises human cells that mimic neuronal cells affected in AD, including immortalized neuronal cells and iPS cells. Preferably, said cells are provided with the compound to be tested prior to addition of the substrate of the invention. Preferably, said cells are subsequently lysed and mixed with said substrate.

In a preferred embodiment, said changed enzyme activity comprises an increased enzyme activity.

In another embodiment, cells are provided with the compound to be tested prior to addition of the substrate of the invention. Preferably, said cells are subsequently lysed and mixed with oligomerized substrate of the invention.

In a preferred embodiment, the substrates according to the invention are suitably used to screen for compounds that improve the capability of cells in the central nervous system (neurons, glia cells) to degrade Aβ, e.g. by increasing endogenous IDE mRNA or protein levels, or IDE activity towards Aβ. As shown in Figure 1 (A), the effect on degradation of qAβ40, amount of aggregation, and solubilization back to monomeric qAβ, are all reflected in the slope of the fluorescence curve (higher slope, is more efficient degradation) and the height of the plateau (a higher plateau is less aggregated/oligomeric qAβ40).

The method of the invention may be performed by incubating said cells, preferably human neuronal cells, with small molecule compounds or an siRNA library for e.g. 24, 48 or 72 hours, and either generate cell lysates, for cytosolic fractions, or use the intact cells to detect improved extracellular Aβ degradation. Said cells or lysates may then be incubated with the substrate of the invention and the efficiency of degradation in time of said substrate may be determined using a fluorescence plate reader. An increase in degradation of said substrate may occur when IDE activity is improved at the transcription/translational/post-translational level, or other yet unknown enzymes are activated that can target said substrate, or when chaperones are induced that can keep the substrate of the invention monomeric in order to improve degradation by IDE. As a control, a peptide comprising a quencher and a fluochrome, but not comprising a recognition site for IDE may suitably be used in order to identify specific compounds that improve degradation of a substrate of the invention only.

In an alternative embodiment, the substrate of the invention is allowed to oligomerize and subsequently added to cells or cell lysates and treated with compounds, as described above. An increase in fluorescence will indicate that oligomers are degraded, either as oligomers or due to the upregulation of e.g. chaperones that can dissolve the oligomers of the substrate of the invention to monomers, which are then subsequently degraded.

### EXAMPLE 1

### Summary

Alzheimer's disease is hallmarked by amyloid β peptides accumulation and aggregation in extracellular plaques, preceded by intracellular accumulation. We examined whether intracellular amyloid β can be cleared by cytosolic peptidases, and whether this capacity is affected during progression of sporadic Alzheimer's disease in humans and in the commonly used APPswePS1dE9 and 3xTg-AD mouse models. A quenched amyloid β peptide that becomes fluorescent upon degradation was used to screen for amyloid β-degrading cytoplasmic peptidases cleaving the aggregation-prone KLVFF region of the peptide. In addition, this quenched peptide was used to analyze amyloid β degrading capacity in the hippocampus of sporadic Alzheimer's disease patients with different Braak stages as well as APPswePS1dE9 and 3xTg-AD mice. Insulin degrading enzyme (IDE) was found to be the main peptidase that degrades cytoplasmic, monomeric amyloid β. Oligomerisation of amyloid β prevents its clearance by IDE. Intriguingly, the amyloid β degrading capacity decreases already during the earliest Braak stages of sporadic Alzheimer's disease, and this decline correlates with IDE protein levels, but not with mRNA levels. This suggests that decreased IDE levels could contribute to early sporadic Alzheimer's disease. In contrast to the human data, the commonly used APPswePS1dE9 and 3xTg-AD mouse models do not show altered amyloid β degradation and IDE levels with Alzheimer's disease progression, raising doubts whether mouse models that overproduce amyloid β peptides are representative for human sporadic Alzheimer's disease.
**Abbreviations:** Aβ= Amyloid-beta peptide, AD= Alzheimer's disease, sAD= Sporadic Alzheimer's disease, fAD= Familial Alzheimer's disease IDE= Insulin degrading enzyme

### Introduction

Alzheimer's disease (AD) is hallmarked by extracellular deposits of amyloid-β (Aβ) peptides in plaques and intracellular neurofibrillary tangles containing hyperphosphorylated tau. According to the amyloid-cascade hypothesis, Aβ accumulation in the brain drives further AD pathogenesis, such as tau aggregation, synaptic dysfunction, and eventually neuronal death. The progression of AD is classified by different Braak stages and amyloid scores (Braak & Braak, 1991), reflecting the distribution of plaques and tangles in well-defined brain areas. Aβ peptides are generated from the transmembrane amyloid precursor protein (APP) by β-secretase and γ-secretase in organelles including the endoplasmic reticulum, Golgi-apparatus and the endosomal-lysosomal pathway. Predominantly Aβ peptides of 40 (Aβ₄₀) or 42 amino acids (Aβ₄₂) are generated, with Aβ₄₂ being more aggregation-prone (Thinakaran & Koo, 2008). While extracellular Aβ aggregation has long been considered as the primary cause of AD, intracellular Aβ accumulation is detected in neurons prior to the appearance of extracellular deposits and is associated with cytotoxicity, dysfunction of organelles and neurodegeneration. Translocation of Aβ peptides into the cytoplasm could occur via various routes, including transport of ER-generated Aβ peptides into the cytoplasm via systems related to ER associated degradation (ERAD), passive leakage of Aβ along the secretory pathway, or by membrane permeability of lysosomes containing internalized Aβ.

While overproduction of Aβ underlies the rare autosomal dominant familial AD (fAD), far more common is late-onset sporadic AD (sAD), thought to be caused by decreased clearance of the Aβ peptide. Several proteases are able to degrade Aβ and especially IDE, also named insulysin, is extensively described for its role as an Aβ degrading enzyme (Kurochkin & Goto, 1994;). IDE is mainly cytoplasmic, but also has a neuronal transmembrane isoform, is found in peroxisomes and can be secreted via exosomes by microglia and astrocytes. Knock down of IDE levels in HeLa cells was shown to result in accumulation of cytoplasmic Aβ peptides after they were transported to the cytoplasm via ERAD, subsequently capable of inducing cell death.

Our study aims at elucidating the role for peptidases in cytoplasmic Aβ clearance during different stages in the development of AD, in both human post-mortem brains and brains of the commonly used APPswePS1dE9 and 3xTg-AD mouse models. Using a quenched Aβ peptide that becomes fluorescent upon cleavage inside the KLVFF region critical for Aβ peptide aggregation, we confirmed IDE to be the main peptidase that degrades cytoplasmic Aβ. More important, we found that the capacity to degrade Aβ is already dramatically decreased during the earliest stages of sAD in humans. This decline correlated with IDE protein levels but not RNA levels. Interestingly, these changes in IDE levels and Aβ degradation were not observed in the APPswePS1dE9 and 3xTg-AD mouse models, raising doubts whether mouse models that overproduce amyloid β peptides mimic the alterations in Aβ clearance in human sAD.

### Results

### IDE is the main cytoplasmic peptidase degrading the monomeric Aβ peptide

We developed a sensitive Aβ degradation assay by generating a quenched Aβ₄₀ peptide containing a small fluorescein group and a quenching dabcyl group (Reits et al., 2004) flanking the KLVFF sequence present in the middle of the Aβ peptides that is a critical region for aggregation (Fig. 1A) (Tjernberg et al., 1996). A quenched Aβ₄₂ peptide was difficult to synthesize as the peptide was too aggregation-prone during synthesis, preventing efficient labeling with the small fluorophore. The quenched Aβ peptide (qAβ) only becomes fluorescent upon separation of quencher and fluorophore, hence after degradation of Aβ. The qAβ peptides were added to cytoplasmic fractions of mildly lysed HEK293 cells, thereby preventing contamination with extracellular membrane-bound or lysosomal proteases, resulting in an increase in fluorescent signal (Fig. 1B). To identify which proteases play a role in this Aβ clearance machinery, different protease inhibitors were added to the cytosolic fractions prior to adding the qAβ. The proteasome inhibitor MG132 did not show any effect on Aβ degradation, nor did inhibition of cystein peptidases (E64), bestatin-sensitive aminopeptidases, serine proteases (PMSF), aspartyl proteases (pepstatin), puromycin-sensitive aminopeptidase (PAQ22), tripeptidyl-peptidase (butabindide, AAF-CMK), thimet oligopeptidase (Cpp-AAF-pNa) or nardilysin (amasatin). However, inhibiting metalloproteases by adding phenantroline significantly decreased the rate of qAβ degradation. Specific inhibition of the metallopeptidase IDE by using bacitracin reduced Aβ degradation (Fig. 1C) and since no additional decrease was observed upon combining phenantroline and bacitracin, this indicates that IDE is the main cytoplasmic protease responsible for efficient Aβ degradation.

Since most peptidases can only target monomeric Aβ, inhibition of IDE and the resulting decrease in degradation rate of qAβ, could accelerate aggregation of the non-degraded qAβ. Therefore, the effect of Aβ oligomerisation on degradation rates was investigated. The qAβ peptide was pre-incubated at 37 °C to allow oligomerization for 2-6 hours before addition to the cytosolic fraction. Reduced fluorescent levels were found with pre-incubated qAβ peptides (Fig. 1D), suggesting that aggregation of qAβ peptides prevents its degradation. To exclude that this effect was due to clogging of IDE rather than an inability to degrade oligomeric forms of qAβ, monomeric or 2 hours pre-incubated oligomeric qAβ was added to cytosolic fractions. When no degradation occurred anymore, indicated by reaching the plateau level of fluorescence, a second pool of only monomeric qAβ peptides was added to the same lysates. These peptides were equally efficiently degraded (Fig. 1E), demonstrating that IDE did not become clogged by oligomeric qAβ. Aggregation of qAβ was confirmed by electron microscopy, showing increased oligomerisation in time, comparable to unmodified Aβ₄₂ (Fig. 1F).

### Aβ peptides are less efficiently degraded by IDE with sAD development

Since the hippocampus is highly susceptible to Aβ accumulation, we examined whether the ability to degrade Aβ is affected in sporadic (sAD) using human hippocampal tissue of post-mortem brains with varying Braak stages. Included were Braak 0 (control, no AD) and Braak I-VI. Information regarding post-mortem samples is listed in Table 1. When the ability to degrade qAβ was determined for each Braak stage (Fig. 2A, solid bars), a strong decrease in qAβ degradation rate was already observed in Braak stage I (t-test, two-tailed, p=0.03), which further decreased during the progression to Braak stage II (t-test, two-tailed, p=0.01). Intriguingly, in Braak stage III the ability to degrade qAβ was partially restored, only to decrease again in Braak stage IV (t-test, two-tailed, p=0.02) and further decrease as a function of progression of Braak stage (Braak V: t-test, two-tailed, p=0.0005, Braak VI: t-test, two-tailed, p=0.000, t-test). In Braak VI, qAβ degradation rate decreased with more than 65% compared to Braak 0. When bacitracin was added to hippocampal lysates prior to addition of the qAβ, the ability to degrade qAβ was strongly reduced in all Braak stages upon IDE inhibition (Fig. 2A, dotted bars). This indicates that IDE is also the main protease responsible for Aβ clearance in human hippocampal lysates and suggests that IDE is impaired in sAD. Also oligomerized Aβ was less well degraded with increasing Braak stage (Fig. 2B). AD progression can also be classified by plaque distribution in well defined brain areas giving a particular amyloid score, ranging from O (no plaques) to C. Similar to increasing Braak stages, the rate of qAβ degradation decreased with increasing amyloid score, being significant for hippocampal fractions of brains classified with amyloid score C (t-test, two-tailed, p=0.017) (Fig. 2C). Whereas qAβ degradation rate is significantly negatively correlated with amyloid score and Braak stages (respectively; Pearson's correlation coefficient r=-0.499, p=0.000 and r=-0.469, p=0.000), qAβ degradation rate was not significantly correlated with postmortem delay, sex, age, ApoE genotype. When combining Braak stages and amyloid scores, the correlation with qAβ degradation was even stronger (Pearson's correlation coefficient r=-0.583, p=0.000) (Table 2).

To examine the reduced qAβ degradation in sAD in more detail, we examined IDE protein and mRNA levels, since IDE was found to be the main peptidase degrading the qAβ. Strikingly, a strong decrease in IDE protein levels was observed already in Braak stage I, which further decreased during the progression to Braak stage II, in parallel with the qAβ degradation rate. Similarly, IDE protein levels partially restored in Braak stage III, and decreased again as a function of progression of Braak stage from Braak stage IV onwards (Fig. 2D), indicating that decreased IDE protein levels were directly linked to impaired qAβ degradation and development of sAD. In contrast, IDE mRNA did not correlate with IDE proteins levels and qAβ degradation; mRNA levels did not change with Braak stage and were even upregulated with amyloid score, being significant for amyloid score B (t-test, two-tailed, p=0.03).

### Aβ peptides are efficiently degraded in all stages of AD in the APPswePS1dE9 mice

Several genetically modified mouse models have been generated that mimic human AD pathology, including the APPswePS1dE9 double transgenic mice which co-express APP695 with the Swedish mutation (K594M/N595L) and the human exon-9-deleted variant of PS1 (PS1-dE9). The APPswe mutation is a favorable substrate for β-secretase, whereas the PS1dE9 mutation alters γ-secretase cleavage, thereby promoting overproduction of Aβ₄₂. Consequentially, APPswePS1dE9 mice show increased Aβ₄₂ production accompanied by plaque pathology in the brain, becoming evident at the age of 6 months (Jankowsky et al., 2004). APPswePS1dE9 mice are commonly used in AD research for behavioral tests and studying the molecular mechanisms in plaque progression. To examine whether the APPswePS1dE9 mice show a similar decrease in Aβ degradation rate with progression of AD, qAβ degradation was measured using hippocampal lysates of APPswePS1dE9 mice at the age of 3 months (no plaque pathology), 9 months (starting plaque pathology) and 18 months (severe plaque pathology) (Kamphuis et al., 2012b) in parallel with wild-type age matched controls (n=3 per age group). Strikingly, hippocampal lysates of the APPswePS1dE9 mice at all stages of AD showed equal qAβ degradation rates as those of age matched wild-type controls (Fig. 3A), which is in clear contrast with human sAD. As qAβ degradation was equally decreased in all lysates upon inhibition of IDE (Fig. 3A), this suggests that IDE is also the main peptidase responsible for Aβ degradation in APPswePS1dE9 mice. Just as for the human hippocampal lysates, we measured IDE protein levels in the hippocampal lysates of the APPswedE9 mice at all stages of AD. However, in clear contrast with the human data, neither IDE protein (Fig. 3B) nor mRNA levels (data not shown) change during AD progression in mice. Unaltered IDE protein levels could explain why APPswePS1 dE9 mice do not show a decrease in Aβ degrading abilities with AD development and indicates that in APPswePS1dE9 mice plaque formation is mainly the result of an increase in Aβ production.

### Aβ peptides are efficiently degraded in all stages of AD in the 3xTg-AD mice

The triple transgenic mouse model (3xTg-AD) is the first mouse model to develop both plaque and tangle pathology in AD-relevant brain regions and is often used to study human AD pathology. The 3xTg-AD mice harbor three mutant genes; APP695 with the Swedish mutation (K670M/N671L), PS1_{M146V} and tau_{P301L}, together promoting the overproduction of Aβ₄₂ and tau. Despite equivalent overexpression of human APP and tau, intraneuronal Aβ accumulation and Aβ deposition precede tangle pathology and both pathologies develop in an age-dependent manner with a temporal and regional specific profile that closely mimics their development in human AD brain. To examine whether the 3xTg-AD mice mimic the decreased Aβ degradation rate with progression of AD as observed in the hippocampus of human sAD, we measured qAβ degradation rate in hippocampal lysates of 3xTg-AD mice at the age of 3 months (no AD pathology), 9 months (starting AD pathology), 15 months (severe AD pathology), in parallel with wild-type age matched controls (n=3 per group). Interestingly, just like we observed in the APPswePS1dE9 mouse model, and in clear contrast with the human data, also the 3xTg-AD mice do not show any change in Aβ degradation capabilities with AD progression; at all stages of AD the 3xTg-AD mice show equal qAβ degradation rates as those of age matched wild-type controls (Fig. 4A). Inhibition of IDE resulted in decreased qAβ degradation, again showing IDE as the main peptidase degrading Aβ. As expected from these results, IDE protein levels were equal at all stages of AD (Fig. 4B).

### Discussion

The present study shows IDE as the main peptidase that degrades cytoplasmic monomeric Aβ. In addition we provide evidence that IDE less efficiently degrades oligomeric Aβ and deficiencies in IDE activity might therefore lead to less Aβ degradation and subsequent Aβ oligomerisation and accumulation. The present study is the first showing that the capacity to degrade Aβ is already decreased dramatically in Braak stage I and II of sAD, in contrast to non demented controls that are efficiently degrading Aβ. Interestingly, degradation of Aβ is partly restored in stage III and then again decreased until stage VI. This decrease in Aβ degradation is in parallel with IDE protein levels, including the temporal revival in stage III. It is tempting to speculate that IDE levels are increased as a rescue mechanism to cope with AD pathology, but might also result from changes in IDE secretion. This pattern has not been described before, probably because previous studies were grouping different-staged AD patients together, and because peptidase activities were never investigated for the separate Braak stages. However, our study includes a relatively high number of individuals covering all stages of AD. It seems that the decrease in IDE activity is not the result of a general decrease in peptidase activities, since other peptidases did not show the same pattern of decreasing activity across the Braak stages (data not shown) (Reits et al., 2004). Also, it is unlikely that the observed pattern was caused by competition of the qAβ with increased endogenous Aβ for degradation by IDE, as endogenous Aβ levels were are a 10.000 fold lower than the added qAβ levels (Roher et al., 2009). In addition, increased endogenous Aβ levels are found in both hippocampal tissue of AD patients (Matsui et al., 2007), as well as in the APPswePS1dE9 and 3xTg-AD mouse models (Together, these observations exclude a significant effect of competition or accelerated aggregation of endogenous Aβ levels as an alternative explanation for the correlation of IDE levels and Aβ degradation in sAD.

The mechanisms underlying the partially recovery of IDE protein levels and Aβ degradation at Braak stage III remains to be established. It should be noted that stage III is also the stage at which large transcriptional changes occur in human AD brain. IDE mRNA levels were not changed with increasing Braak stage and thus did not correspond to altered protein levels of IDE. This suggests that changes in IDE protein levels that were observed in AD brain are caused by changes in IDE half-life or secretion. Interestingly, a similar decrease and temporal increase was observed when measuring qAβ degradation in the cerebrospinal fluid of sAD patients at different stages, indicating that the ability to degrade Aβ may be globally affected in the central nervous system. Since changes in Aβ degradation can already be monitored during the earliest stages of the disease, this would provide possibilities to diagnose AD before the appearance of symptoms and the development of plaque pathology.

The present study also provides evidence that the commonly used mouse models APPswePS1dE9 and the 3xTg-AD mice, both showing increased Aβ₄₂ production accompanied by dense-core plaque pathology in the brain, do not mimic the alterations in Aβ clearance observed in human sAD. Neither changes in Aβ degradation with AD development nor changes in IDE levels were observed in these mice. A possible explanation for the observed difference between mice and men would be the genetic background of these mice where overproduction of the amyloid β peptide (Aβ) is the primary cause for AD, in contrast to sAD where decreased clearance of Aβ seems to be the primary mechanism underlying the development of the disease, also confirming previous data. Also the present study is supporting this view by showing altered levels of IDE in human hippocampal tissue, but not in APPswePS1dE9 and 3xTg-AD mouse hippocampi. Furthermore, these data show that aging per se does not affect the IDE activity and IDE levels, since aging wild-type mice did also not show decreased Aβ degradation. The comparison of our results from mice and human show that mice data should be considered with caution, as they seem to be not representative for human sAD.

### Materials and methods

### Human subjects

Human post-mortem tissue was obtained from the Netherlands Brain Bank (NBB; Amsterdam, The Netherlands). The brain donors have given informed consent for using the tissue and for accessing the extensive neuropathological and clinical information for scientific research, in compliance with ethical and legal guidelines (Huitinga et al., 2008). Included in this study were Braak stage 0 (n=9) and I (n=10), II (n=9), III (n=10), IV (n=10), V (n=10) and VI (n=10). Samples were matched as closely as possible for age, sex, post-mortem interval, pH-CSF and ApoE genotype. More detailed donor information is presented in Table 1.

### Animals

Double APPswePS1dE9 transgenic mice expressed chimeric mouse/human APP containing the K959N/M596L Swedish mutation and human PS1 variant carrying the Exon 9 deletion both driven by mouse prion promotor elements, directing the expression to neurons (Jankowsky et al., 2004). For details see The Jackson Laboratory [strain B6C3-Tg(APPswe, PSEN1-dE9)85Dbo/J; stock number 004462; http://jaxmice.jax.org/]. AD mice were maintained as hemizygous and crossed with wild-type C57BL/6. Genotyping was performed by real-time PCR assays specific for the two transgenes and the prion promoter. The 3xTg-AD line was originally generated by co-microinjection of human APP (K670M/N671L) and tau (P301L) transgenes under the control of the Thy 1.2 promotor into mutant PS-1 (M146V) knock-in mice (Kamphuis et al. (2012a). For details see The Jackson Laboratory [strain B6;129-Psen1_{tm1Mpm}Tg(APPswe,tauP301L)1Lfa/Mmjax; stock number 004807; http://jaxmice.jax.org/]. Wild-type littermates served as controls for the AD animals. All animals were housed under standard conditions with access to water and food *ad libitum.* Animal handling and experimental procedures were reviewed and approved by the ethical committee for animal care and use of experimental animals of the Royal Netherlands Academy for Arts and Sciences, acting in accordance with the European Community Council directive of November 24, 1986 (86/609/EEC). All efforts were made to minimize suffering and number of animals used for the study presented here. 9 APPswePS1dE9 mice and 9 wild-type mice were used, each divided in age groups 3 months (n=3), 6 months (n=3) and 18 months (n=3). Also, 9 3xTg-Ad mice and again 9 wild-type mice were used, each divided in age groups 3 months (n=3), 6 months (n=3) en 15 months (n=3).

### Protein isolation

HEK cells were lysed in 25 µM digitonin (Sigma) in KMH buffer (110 mM KAc, 2 mM MgAc and 20 mM Hepes-KOH, pH 7.2). Human and mouse brain hippocampal tissue was lysed in ± 400 µl (adjusted to the amount of tissue) of homogenization buffer (50 mM Tris/HCl pH 7.5, 250 mM sucrose, 5 mM MgCl₂ and freshly added 2 mM ATP, 1 mM DTT and 0.025 % digitonin (Sigma)). Tissue was homogenized on ice in homogenization buffer using a homogenizer. Cell lysates were incubated for 30 minutes on ice and centrifuged for 15 minutes at 14.000 rpm at 4 °C.

### Quenched Aβ peptide degradation assays

### Peptide synthesis

An Aβ₄₀ peptide DAEFRHDSGYE(**q**)HHQKLVFFA(**f**)DVGSNKGAIIGLMVGGVV containing a fluorophore and quencher was synthesized as described before (Reits et al., 2004), introducing a fluorescein (f) at the cysteine (position 22) and a quenching dabcyl group (q) at the lysine position 12.

### Quenched Aβ peptide degradation assay

HFIP-treated aliquots of Aβ₄₀ were resuspended in DMSO followed by sonication for 10 minutes, immediately before use. 75 ng (340 nM) of quenched Aβ₄₀ was added to 5 µg protein from the cell lysates in KMH buffer (110 mM KAc, 2 mM MgAc and 20 mM Hepes-KOH, pH 7.2) to a total volume of 50 µl. Protease inhibitors (100 µM 1,10-phenanthroline, 200 µM bacitracin, 100 µM E64, 20 µM MG-132, 100 µM puromycin, 100 µM PMSF, 200 µM pepstatin A (all Sigma), 100 µM AAF-CMK, 100 µM amastatin or 50 µM bestatin (Enzo lifesciences AG), 50 µM PAQ-22 (Wako Chemicals), 100 µM Cpp-AAF-pNa (InstruChem), 50 µM PAQ-22 (SopaChem) and 100 µM butabindide (Tocris Bioscience)) were added to the cell lysates and incubated for 30 minutes at 4 °C. Degradation of the peptide was analyzed at 37°C using the FLUOstar OPTIMA (BMG Labtec.).

### Electron microscopy

Aβ peptide preparations were adsorbed on 300-mesh formar/copper grids for 2 minutes and excess fluid was filtered off. Upon staining with 2.5% uranyl acetate for 2 minutes, grids were analyzed with a Fei technai-12 G2 transmission electron microscope.

### RNA isolation and quantitative PCR

RNA from human and mouse hippocampal tissue was isolated using Trizol (Invitrogen) and an overnight precipitation in isopropanol. Total RNA (1.0 µg) was DNAse I treated and used to generate cDNA (QuantiTect Reverse Transcription Kit Qiagen) using oligo-dT and random hexamer primers. The reverse transcriptase reaction was incubated at 42 °C for 30 minutes. The resulting cDNA was diluted 1:20 and served as a template in realt-time qPCR assays. Real-time qPCR (SYBR® Green PCR Master Mix; Applied Biosystems) for IDE was performed using forward primer
GGACAGGTTTGCGCAGTTTT (SEQ ID NO:8) and reverse primer
ACAGCGTTCACCTCTCTGTCTTT (SEQ ID NO:9). Expression levels of human AD samples were normalized against a selection of 10 reference genes (GAPDH, ACTB, PPIA, UBE2D2, EEF1A, RPS27A, AARS, XPNPEP1, RPLP0, IPO8) based on a geNorm analysis. The normalization factor was the geomean of the 10 reference genes. Samples with a RIN value below 5.0 were excluded from analysis based on a poor correlation with the normalization factor. Expression levels of mouse AD samples were normalized against a selection of 4 reference genes (GAPDH, HPRT, ACTB and 18S rRNA). The geomean of the reference gene levels was used to normalize the assessed transcript levels of IDE.

### Western blotting

Equal protein amounts obtained from the hippocampal cell lysates were separated on 7.5 % SDS-PAGE gels. After electrophoresis, proteins were transferred either onto a 0.45 µm pore size PVDF membrane filter (Schleicher & Schuell). Blots were blocked in 5% dry milk in TBS and incubated with the primary antibodies against IDE (1:500; Abcam) and β-actin (1:500; Abcam) and subsequently with secondary antibodies IRDye 680 or IRDye 800 (1:15.000; LI-COR Biosciences). Signal was detected using the Odyssey imaging system (Licor).

**Table 1.**

| | Amyloid score | Age | Sex | PMD | pH | BW |
|---|---|---|---|---|---|---|
| Br 0 (n=9) | 60-2A-1B | 60.1 ± 6.0 | 2F-7M | 7.2 ± 3.1 | 5.9 ± 2.2 | 1282 ± 177 |
| Br I (n=10) | 40-3A-1B-1C-1NA | 81.8 ± 9.4 | 5F-5M | 6.7 ± 1.9 | 6.6 ± 0.3 | 1239 ± 144 |
| Br II (n=9) | 3O-2B-2C-2NA | 84.8 ± 6.8 | 6F-3M | 6.0 ± 0.9 | 6.5 ± 0.2 | 1213 ± 99 |
| Br III (n=10) | 1O-1A-2B-2C-4NA | 85.2 ± 6.6 | 6F-4M | 5.2 ± 2.1 | 6.5 ± 0.3 | 1235 ± 151 |
| BrIV (n=10) | 1B-7C-2NA | 85.2 ± 5.6 | 5F-5M | 5.5 ± 1.8 | 6.6 ± 0.2 | 1176 ± 163 |
| Br V (n=10) | 2B-7C-1NA | 81.2 ± 9.4 | 5F-5M | 5.8 ± 1.4 | 6.4 ± 0.2 | 1138 ± 95 |
| Br VI (n=10) | 1B-6C-3NA | 77.5 ± 11.4 | 5F-5M | 5.6 ± 1.7 | 6.5 ± 0.2 | 1072 ± 143 |

| | | | | | | |
|---|---|---|---|---|---|---|
| **Detailed donor information.** BW= Brain weight (grams), NA= Not available, PMD= Post-mortem delay (hours). | | | | | | |

**Table 2.**

| | Pearson's correlation | P-value |
|---|---|---|
| Braak stage | -0.469 | 0.000 |
| Amyloid score | -0.499 | 0.000 |
| Braak stage * Amyloid score | -0.583 | 0.000 |
| Postmortem delay (h) | 0.009 | 0.941 |
| Age at death | -0.153 | 0.213 |
| Sex | -0.133 | 0.281 |
| ApoE | -0.151 | 0.243 |

**Correlation patient variables and qAβ degradation.** Pearson's correlation values and significance for correlation between qAβ degradation and Braak stage, Amyloid score, Braak stage*Amyloid score, Postmortem delay, Age at death, Sex and ApoE.

### References

Braak H, Braak E (1991) Neuropathological stageing of Alzheimer-related changes. Acta Neuropathol 82, 239-259.
Huitinga I, Rademaker M, Klioueva N (2008) The art of brain banking in Europe: ethical, legal and practical guidelines for donor recruitment, tissue handling and tissue distribution. J Neural Transm 115, 1715.
Jankowsky JL, Fadale DJ, Anderson J, Xu GM, Gonzales V, Jenkins NA, Copeland NG, Lee MK, Younkin LH, Wagner SL (2004) Mutant presenilins specifically elevate the levels of the 42 residue β-amyloid peptide in vivo: evidence for augmentation of a 42-specific γ secretase. Human Mol Genet 13, 159-170.
Kamphuis W, Mamber C, Moeton M, Kooijman L, Sluijs JA, Jansen AHP, Verveer M, de Groot LR, Smith VD, Rangarajan S (2012a) GFAP Isoforms in Adult Mouse Brain with a Focus on Neurogenic Astrocytes and Reactive Astrogliosis in Mouse Models of Alzheimer Disease. Public Library of Science 7, e42823.
Kamphuis W, Orre M, Kooijman L, Dahmen M, Hol EM (2012b) Differential cell proliferation in the cortex of the appsweps1de9 alzheimer's disease mouse model. Glia
Kurochkin IV, Goto S (1994) Alzheimer's β-amyloid peptide specifically interacts with and is degraded by insulin degrading enzyme. FEBS Lett 345, 33-37.
Reits E, Neijssen J, Herberts C, Benckhuijsen W, Janssen L, Drijfhout JW, Neefjes J (2004) A major role for TPPII in trimming proteasomal degradation products for MHC class I antigen presentation. Immunity 20, 495-506.
Roher AE, Esh CL, Kokjohn TA, Castaño EM, Van Vickle GD, Kalback WM, Patton RL, Luehrs DC, Daugs ID, Kuo Y, Emmerling MR, Soares H, Quinn JF, Kaye J, Connor DJ, Silverberg NB, Adler CH, Seward JD, Beach TG, Sabbagh MN (2009) Amyloid beta peptides in human plasma and tissues and their significance for Alzheimer's disease. Alzheimer's and Dementia 5, 18-29.
Thinakaran G, Koo EH (2008) Amyloid precursor protein trafficking, processing, and function. J Biol Chem 283, 29615-29619.
Tjernberg LO, Naslund J, Lindqvist F, Johansson J, Karlstrom AR, Thyberg J, Terenius L, Nordstedt C (1996) Arrest of beta-amyloid fibril formation by a pentapeptide ligand. J Biol Chem 271, 8545-8548.

### EXAMPLE 2

### Degradation of the 12-mer HQKLVFFACDCB peptide

To proof that shorter variants of the substrates of the invention may also be used as a suitable substrate to measure IDE activity, we analyzed the degradation of a substrate having the amino acid sequence HQKLVFFACDBC, which contains the KLVFF region flanked by a quenched and fluorophore. In addition, we examined the effect of bacitracine, the inhibitor for IDE, on the degradation of this 12-mer peptide (see Figure 5), as well as its ability to aggregate and form oligomers. When monomeric forms of this 12-mer peptide were added to SH-SY5Y cell lysates, degradation could be observed (solid line), that was inhibited by addition of bacitracine (dotted line), demonstrating that the peptide is mainly cleaved by IDE. Furthermore, incubation of the peptide at 37°C showed aggregation leading to a lower plateau level (dashed line). These results demonstrate that also a shorter variant containing the KLVFF region is mainly a substrate for IDE and has aggregation prone properties.

The 12-mer substrate having the amino acid sequence HQKLVFFACDCB (SEQ ID NO:10) containing a fluorophore and quencher was synthesized as described before (Reits et al., 2004), introducing a fluorescein at the cysteine (position 11) and a quenching dabcyl group at the lysine (position 3). The substrate was sonificated immediately before use and added to 5 µg cell lysate. KMH buffer (110 mM KAc, 2 mM MgAc and 20 mM Hepes-KOH, pH 7.2) was added to a total volume of 50 µl. Degradation of the substrate was analyzed at 37°C using the FLUOstar OPTIMA (BMG Labtec.).

### EXAMPLE 3

### Degradation of the substrate according to the invention in human Cerebrospinal fluid.

To proof that the substrates of the invention are suitable for diagnostic purposes, we determined the IDE activity in cerebrospinal fluid (CSF) of healthy controls and Alzheimer Disease patients in different Braak stages, using substrates of the invention. Interestingly, changes in degradation of the substrate were already observed in Braak stage I and was significantly changed in Braak stage II (p<0.05) (Figure 6A). Also when Braak stages were pooled, a significant difference was observed between CSF obtained from control and AD (Figure 6B).

### Materials and Methods

Post mortem non-hemolytic ventricular CSF was obtained from healthy controls (N=6) and neuropathologically confirmed AD patients (N=39). Included in this study were subjects in Braak stage 0 (n=6) and I (n=7), II (n=8), IV (n=6), V (n=7) and VI (n=5). HFIP-treated aliquots of qAβ40 were resuspended in DMSO followed by sonication for 10 minutes, immediately before use. 75 ng (340 nM) of quenched Aβ40 was added to 5 µg protein in CSF. KMH buffer (110 mM KAc, 2 mM MgAc and 20 mM Hepes-KOH, pH 7.2) was added to a total volume of 50 µl. Degradation of the substrate was analyzed at 37°C using the FLUOstar OPTIMA (BMG Labtec.).

## Claims

1. A substrate for detecting the activity of IDE, of formula F-P-Q
- wherein P represents a peptide comprising at least one cleavage site for Insulin Degrading Enzyme (IDE),
- wherein F represents a fluorescent dye which is attached to at least one amino acid residue of P,
- wherein Q represents a quencher substance being able to quench the fluorescent dye F, and being attached to at least one amino acid residue of P, and
- whereby F and Q are attached to amino acids residues on different sides of the cleavage site of P,
with the proviso that there is no cleavage site for Angiotensin Converting Enzyme (ACE) present in said peptide between the amino acids to which F and Q are attached.

2. Substrate according to claim 1, selected from the group consisting of:
a) a peptide having the amino acid sequence
DAEFRHDSGYEKHHQKLVFFACDVGSNKGAIIGLMVGGVV (SEQ ID NO: 1),
or
b) a functional fragment thereof,
c) a homologue thereof, and
d) a chemical derivative of any of the peptide, functional fragment and analogue as defined in a)-c).

3. Substrate according to claim 1 or 2, wherein said peptide has an amino acid sequence which is at least 70% homologous to SEQ ID NO: 1.

4. Substrate according to anyone of claims 1-4, wherein said peptide comprises the amino acid sequence selected from the group consisting of: VFF, LVFF, KLVFF, QKLVFF, HQKLVFF, QKLVFFA, HHQKLVFF and HQKLVFFA (SEQ ID NO:2).

5. Substrate according to anyone of claims 1-4, wherein said peptide comprises at least 10, more preferably at least 11, 12, 13, 14, 15, 16, 17, 18, 19, 20, 21, 22, 23, 24 or 25 amino acids.

6. Substrate according to anyone of claims 1-5, comprising at its termini a D amino acid, or a chemical modification of a terminal residue which protects against degradation by an exopeptidase.

7. Substrate according to anyone of claims 1-6, having the amino acid sequence selected from the group consisting of SGYEKHHQKLVFFACDVGS (SEQ ID NO:3), YEKHHQKLVFFACDV (SEQ ID NO:4), EKHHQKLVFFACD (SEQ ID NO:5), RHDSGYEKHHQKLVFFACDVGSNKG (SEQ ID NO:6) and HQKLVFFACDCB (SEQ ID NO:10).

8. Substrate according to anyone of claims 1-7, wherein said quencher substance is positioned vis-à-vis said fluorescent dye, such that there the distance between said quencher substance and said fluorescent dye is between 6-12 amino acids.

9. Substrate according to anyone of claims 1-8, wherein said quencher substance and said fluorescent dye are attached to the amino acids of position 12 and 22.

10. Method of diagnosis or prognosis of sporadic Alzheimer's disease in a subject, comprising steps of determining the enzyme activity of Insulin-degrading enzyme (IDE) in a biological sample of a subject and diagnosing or prognosticating sporadic Alzheimer's disease based on said enzyme activity of IDE.

11. Method of determining the enzyme activity of IDE, comprising contacting an amount of IDE to a substrate according to any one of claims 1-10, allow said IDE to degrade said substrate, and measuring the fluorescence of said substrate and determining the enzyme activity of IDE based on said fluorescence .

12. Method of determining the enzyme activity of IDE, comprising contacting an amount of IDE to a substrate of formula F-P-Q in the presence of an inhibitor of ACE
- wherein P represents a peptide comprising at least one cleavage site for Insulin Degrading Enzyme (IDE),
- wherein F represents a fluorescent dye which is attached to at least one amino acid residue of P,
- wherein Q represents a quencher substance being able to quench the fluorescent dye F, and being attached to at least one amino acid residue of P, and
- whereby F and Q are attached to amino acids residues on different sides of the cleavage site of P,
allow said IDE to degrade said substrate, and measuring the fluorescence of said substrate and determining the enzyme activity of IDE based on said fluorescence.

13. Method according to claim 10, wherein said enzyme activity of IDE is determined according to the method of claim 11 or 12.

14. Method according to claim 10 or 12, wherein said biological sample is cerebrospinal fluid, blood or serum.

15. Method a of selecting a compound or composition suitable for the treatment of Alzheimer's Disease in a subject comprising steps of:
a) providing a sample comprising IDE, a compound or composition to be tested, and the substrate according to the invention,
b) measuring the fluorescence of said substrate,
c) determining the activity of IDE based on said fluorescence, and
d) selecting the compound or composition based on an increased enzyme activity of IDE compared to a control sample.
